# EUROPEAN PATENT SPECIFICATION

(11) **EP 2 698 118 B1**
(45) Date of publication and mention of the grant of the patent: **04.10.2017**
(21) Application number: 13180373.6
(22) Date of filing: 14.08.2013
(51) Int. Cl.: A61B 17/072, A61F 2/30, A61B 17/00

(54) **Buttress attachment to degradable polymer zones**
Versteifungsbefestigung an abbaubaren Polymerbereichen
Fixation de renfort de zones sur un polymère dégradable

(30) Priority: 15.08.2012 US 201213586261
(43) Date of publication of application: 19.02.2014
(73) Proprietor: Covidien LP, Mansfield, MA 02048 (US)
(72) Inventor: Hodgkinson, Gerald N., Guilford, CT Connecticut 06437 (US)
(74) Representative: Maschio, Antonio

(56) References cited:
- EP-A1- 2 517 637
- EP-A1- 2 630 922
- EP-A2- 2 005 894
- US-A1- 2009 095 791
- US-A1- 2011 278 346

## Description

### BACKGROUND

### 1. Technical field

The present disclosure relates to surgical stapling apparatus including surgical buttresses which can be releasably attached to the surgical stapling apparatus, and in particular, to surgical stapling apparatus having attachment pads made from the same material as the surgical buttress. The attachment pads join the surgical buttress to the surgical stapling apparatus such that the surgical buttress is released upon firing of the surgical stapling apparatus.

### 2. Background of Related Art

Surgical stapling apparatus are employed by surgeons to sequentially or simultaneously apply one or more rows of fasteners, e.g., staples or two-part fasteners, to body tissue for the purpose of joining segments of body tissue together. Such apparatus generally include a pair of jaws or finger-like structures between which the body tissue to be joined is placed. When the stapling apparatus is actuated, or "fired", longitudinally moving firing bars contact staple drive members in one of the jaws. The staple drive members push the surgical staples through the body tissue and into an anvil in the opposite jaw which forms the staples. If tissue is to be removed or separated, a knife blade can be provided in the jaws of the apparatus to cut the tissue between the lines of staples.

A number of surgical stapling apparatus rely on a knife blade cutting off some portion of the surgical buttress to affect release. These methods typically employ a secondary material or mounting structure in addition to the surgical buttress (e.g., sutures) to provide attachment of the surgical buttress to the surgical stapling apparatus. Typically, firing forces are increased with each material that must be transected by the knife blade in order to release the surgical buttress.

It would be desirable to provide a surgical buttress that may be releasably secured to a surgical stapling apparatus without the need for a secondary material or mounting structure, and without the need for a knife blade to cut the buttress and/or secondary material or mounting structure to release the surgical buttress from the surgical stapling apparatus, thereby resulting in the use of fewer materials and lower firing forces Documents US2011278346 and EP2517637 show surgical staplers with buttress attachments. The invention is defined in claim 1. Further embodiments of the invention are defined in the dependent claims.

### SUMMARY

According to one aspect of the present disclosure, an end effector assembly for use with a surgical stapler wherein the end effector comprises a staple cartridge assembly having a tissue contacting surface and an anvil assembly having a tissue contacting surface. A surgical buttress configured and dimensioned to substantially overlie at least a portion of the tissue contacting surface of at least one of the staple cartridge assembly and anvil assembly. A plurality of attachment pads secured to at least one of the tissue contacting surface of the at least one staple cartridge assembly and anvil assembly configured to retain the respective buttress material thereto, wherein the surgical buttress is ultrasonically welded to each of the plurality of attachment pads. The least one of the attachment pads is made from a material selected from the group comprising glycolide, trimethylene carbonate, lactide, caprolactone, and combinations thereof.

In embodiments, the at least one of the attachment pads may be placed into a recess of at least one of the tissue contacting surface of the staple cartridge assembly and anvil assembly.

The staple cartridge assembly and the anvil assembly have a central longitudinally extending slot configured to enable passage of a knife blade therethrough. In embodiments, the at least one attachment pad is positioned near the central longitudinally extending slot.

Each of the staple cartridge assembly and anvil assembly have a distal end such that at least one distal attachment pad is positioned between a first outer edge of each of the staple cartridge assembly and anvil assembly and a distal end of the central longitudinally extending slot. Further, each of the staple cartridge assembly and anvil assembly have a proximal end such that at least one proximal attachment pad is positioned between a first outer edge of each of the staple cartridge assembly and anvil assembly and a proximal end of the central longitudinally extending slot.

In another embodiment, the staple cartridge assembly, the anvil assembly and the surgical buttress are substantially circular, wherein the circular buttress includes an inner portion, an outer portion, and a middle portion extending between the inner portion and the outer portion, and at least on attachment pad secured along the middle portion.

In another aspect of the present disclosure, a staple cartridge for use with a surgical stapling apparatus comprising a cartridge body including a tissue contacting surface defining a plurality of staple retaining slots and a staple disposed within each staple retaining slot of the cartridge body. A surgical buttress configured and dimensioned to substantially overlie at least a portion of the staple retaining slots of the cartridge body. A plurality of attachment pads secured to the tissue contacting surface of the cartridge body configured to retain the respective buttress material thereto, wherein the surgical buttress is ultrasonically welded to each of the plurality of attachment pads.

In yet another aspect of the present disclosure, a surgical stapling apparatus comprising a housing and an end effector being secured to the housing and a staple cartridge assembly having a tissue contacting surface and an anvil assembly having a tissue contacting surface. A surgical buttress configured and dimensioned to substantially overlie at least a portion of the tissue contacting surface of at least one of the staple cartridge assembly and anvil assembly. A plurality of attachment pads secured to at least one of the tissue contacting surface of the at least one staple cartridge assembly and anvil assembly configured to retain the respective buttress material thereto, wherein the surgical buttress is ultrasonically welded to each of the plurality of attachment pads.

### BRIEF DESCRIPTION OF THE DRAWINGS

Various embodiments of the presently disclosed interlocking buttress retention systems are disclosed herein with reference to the drawings, wherein:
FIG. 1 is a perspective view of a surgical stapling apparatus according to an embodiment of the present disclosure;
FIG. 2 is a perspective view, with parts separated, of a staple cartridge assembly of the surgical stapling apparatus of FIG. 1, illustrating a surgical buttress associated therewith;
FIG. 3 is a perspective view, with parts separated, of an anvil assembly of the surgical stapling apparatus of FIG. 1, illustrating a surgical buttress associated therewith;
FIG. 4 is a perspective view of the staple cartridge assembly, illustrating the surgical buttress affixed to a staple cartridge;
FIG. 5 is a perspective view of the anvil assembly, illustrating the surgical buttress affixed to an anvil plate;
FIG. 6 is a perspective view of a distal end of the surgical stapling apparatus of FIG. 1, shown in use positioned about a tissue section;
FIG. 7 is a cross-sectional view taken along line 7-7 of FIG. 6;
FIG. 8 is a perspective view of the stapled and divided tissue section of FIG. 6;
FIG. 9A is a perspective view of an illustrative embodiment of a surgical stapling apparatus in accordance with another embodiment of the present disclosure;
FIG. 9B is a side elevational view, partially broken away, of the surgical stapling apparatus of FIG. 9A;
FIG. 10A is a perspective view of an illustrative embodiment of the staple cartridge assembly of the surgical stapling apparatus of FIG. 9A including a surgical buttress in accordance with an embodiment of the present disclosure;
FIG. 10B is a top plan view of the staple cartridge assembly and surgical buttress illustrated in FIG. 10A;
FIG. 11 is perspective view of an intestinal area of a patient, illustrating a method of positioning an anvil rod and staple cartridge assembly of the surgical stapling apparatus of FIGS. 9A, 9B, and 10 within the intestinal area; and
FIG. 12 is a schematic perspective view of the intestinal area of FIG. 11, illustrating the anvil rod mounted to the surgical stapling apparatus.

### DETAILED DESCRIPTION OF THE EMBODIMENTS

Various exemplary embodiments of the present disclosure are discussed herein below in terms of buttresses for use with surgical stapling apparatus. The buttresses described herein may be used in sealing a wound by approximating the edges of wound tissue between a staple cartridge and an anvil plate of a surgical stapling apparatus which contains at least one surgical buttress. The at least one surgical buttress is joined to the surgical stapling apparatus by at least one attachment pad. The attachment pad is made from the same material as the surgical buttress and positioned between a tissue contacting surface of each of the staple cartridge and anvil plate and the least one surgical buttress. Firing of the surgical stapling apparatus forces legs of at least one staple to pass through an opening on the staple cartridge, the tissue, and into the recesses on the anvil plate to secure the surgical buttress to the tissue, to secure the adjoining tissue to one another, and to seal the tissue. The firing force of the staple impacts the attachment pads to break thereby releasing the surgical buttress from the tissue contacting surface. Thus, the present disclosure describes surgical buttresses, surgical stapling apparatus supporting said surgical buttresses, and methods and mechanisms for using the same.

It should be understood that a variety of surgical stapling apparatus may be utilized with a surgical buttress of the present disclosure. For example, linear stapler configurations may be utilized, such as, for example those including Duet TRS^{™} reloads and staplers with Tri-Staple^{™} technology, available through Covidien, which maintain a principal place of business at 555 Long Wharf Drive, North Haven, CT 06511, and transverse anastomosis staplers, such as, for example, EEA^{™}, CEEA^{™}, GIA^{™}, EndoGIA^{™}, and TA^{™}, also available through Covidien. It should also be appreciated that the principles of the present disclosure are equally applicable to surgical staplers having alternate configurations, such as, for example, end-to-end anastomosis staplers having a circular cartridge and anvil (see, e.g., commonly owned U.S. Patent No. 5,915,616, entitled "Surgical Fastener Applying Apparatus," ; laparoscopic staplers (see, e.g., commonly owned U.S. Pat. Nos. 6,330,965 and 6,241,139, each entitled "Surgical Stapling Apparatus," and transverse anastomosis staplers (see, e.g., commonly owned U.S. Patent Nos. 5,964,394 and 7,334,717, each entitled "Surgical Fastener Applying Apparatus'.

Embodiments of the presently disclosed surgical buttress and surgical stapling apparatus will now be described in detail with reference to the drawing figures wherein like reference numerals identify similar or identical elements. In the following discussion, the terms "proximal" and "trailing" may be employed interchangeably, and should be understood as referring to the portion of a structure that is closer to a clinician during proper use. The terms "distal" and "leading" may also be employed interchangeably, and should be understood as referring to the portion of a structure that is further from the clinician during proper use. As used herein, the term "patient" should be understood as referring to a human subject or other animal, and the term "clinician" should be understood as referring to a doctor, nurse, or other care provider and may include support personnel.

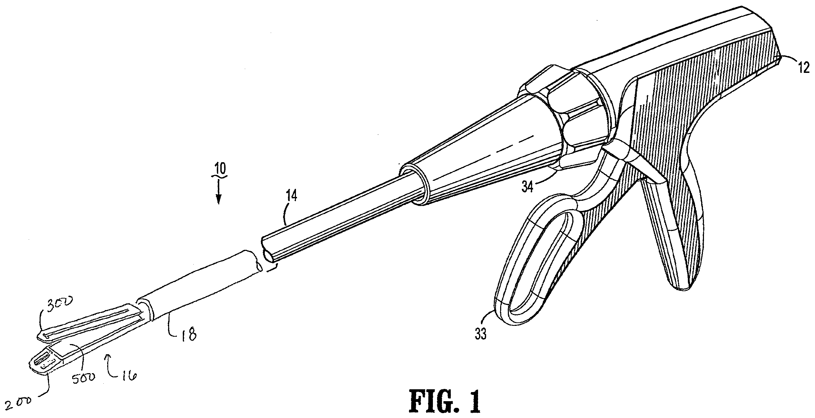

Referring now to FIG. 1, there is disclosed an exemplary surgical stapling apparatus or surgical stapler 10 for use in stapling tissue and applying a buttress material or surgical buttress to the tissue. An example of this type of surgical stapling instrument is disclosed in U.S. Patent No. 7,128,253.

Surgical stapling apparatus 10 generally includes a handle 12 having an elongate tubular member 14 extending distally from handle 12. An end effector assembly 16 is mounted on a distal end 18 of elongate tubular member 14. End effector assembly 16 includes a first jaw or staple cartridge assembly 200 configured to receive a staple cartridge 32 therein and a second jaw or anvil assembly 300. End effector assembly 16 may be permanently affixed to elongate tubular member 14 or may be detachable and thus replaceable with a new end effector assembly 16. It is also contemplated that the staple cartridge can be removable and replaceable. One of staple cartridge assembly 200 and anvil assembly 300 is movably mounted at distal end 18 of end effector assembly 16, and is movable between an open position spaced apart from one another to a closed position substantially adjacent to one another. Anvil assembly 300 supports an anvil plate 302 and is fabricated from a metal material, including and not limited to stainless steel, titanium, titanium alloy, and the like. At least a tissue contacting surface of staple cartridge 32 is fabricated from a material other than metal, including and not limited to plastic, thermoplastic, resin, polycarbonate, and the like.

Surgical stapling apparatus 10 further includes a trigger 33, as seen in FIG. 1, movably mounted on handle 12. Actuation of trigger 33 initially operates to move first jaw and second jaw between the open and the closed positions and simultaneously actuates surgical stapling apparatus 10 to apply lines of staples to tissue. In order to properly orient end effector assembly 16 relative to the tissue to be stapled, surgical stapling apparatus 10 is additionally provided with a rotation knob 34 mounted on handle 12. Rotation of rotation knob 34 relative to handle 12 rotates elongate tubular member 14 and end effector assembly 16 relative to handle 12 so as to properly orient end effector assembly 16 relative to the tissue to be stapled.

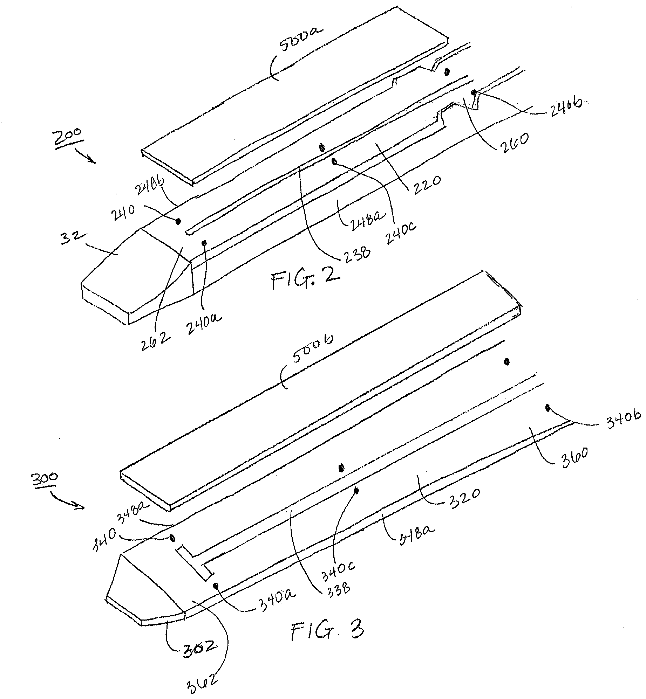

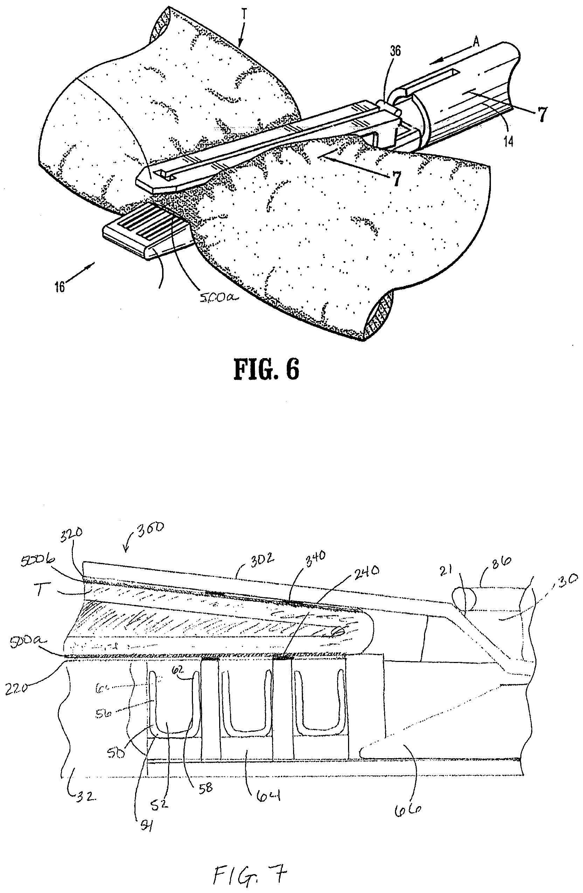

A driver 36, as seen in FIGS. 6 and 7A, is provided to move approximate first jaw or staple cartridge assembly 200 and second jaw or anvil assembly 300 from the open position to the closed position. Driver 36 moves through a longitudinal slot 338 (FIG. 3) formed in the anvil plate 302 of anvil assembly 300. A knife 30 with knife blade 31 is associated with driver 36 to cut tissue captured between staple cartridge assembly 200 and anvil assembly 300 as driver 36 passes through slot 338.

Reference may be made to commonly owned U.S. Patent Nos. 5,915,616, 6,330,965, and 6,241,139, referenced above, for a detailed discussion of the construction and operation of an exemplary surgical stapling apparatus 10.

Staple cartridge assembly 200 and/or anvil assembly 300 may be provided with a surgical buttress 500. Surgical buttress 500 is provided to reinforce and seal the lines of staples applied to tissue by surgical stapling apparatus 10. Surgical buttress 500 may be configured into any shape, size, or dimension suitable to fit any surgical stapling, fastening, or firing apparatus.

Staple cartridge assembly 200 is provided with a cartridge buttress 500a and anvil assembly 300 is provided with an anvil buttress 500b in the manners described in more detail hereinbelow. The buttresses 500a, 500b may be made from any biocompatible natural or synthetic material. The material from which the buttresses 500a, 500b are formed may be bioabsorbable or non-bioabsorbable. It should be understood that any combination of natural, synthetic, bioabsorbable and non-bioabsorbable materials may be used to form the buttress material. The buttresses 500a, 500b may be porous or non-porous, combination of porous and non-porous layers. The non-porous buttresses 500a, 500b may be utilized to retard or prevent tissue ingrowth from surrounding tissues thereby acting as an adhesion barrier and preventing the formation of unwanted scar tissue.

Additional exemplary materials for surgical buttresses 500a, 500b for use with the surgical stapling devices disclosed herein are set forth in commonly assigned U.S. Patent Nos. 5,542,594; 5,908,427; 5,964,774; and 6,045,560, and commonly assigned U.S. Application Publication Nos. 2006/0085034, filed on April 20, 2006; and 2006/0135992, filed on June 22, 2006

As illustrated in the current embodiment and shown in FIG. 2 and 3, surgical buttress 500 is releasably attached to staple cartridge assembly 200 and/or anvil assembly 300 by strategically positioned attachment pads 240, 340 that affix surgical buttresses 500a, 500b to the inwardly facing or tissue contacting surfaces 220, 320 of the staple cartridge 32 and/or the anvil plate 302, as discussed in detail below. The attachment pads 240, 340 are made from the same degradable polymer as the surgical buttresses 500a, 500b. Composing the attachment pads 240, 340 and the surgical buttresses 500a, 500b from the same material allows for easier bonding since the attachment pads 240, 340 and surgical buttresses 500a, 500b have the same melting temperature and chemical properties.

With reference to FIG. 2, cartridge buttress 500a of staple cartridge assembly 200 is operatively secured or adhered to a tissue contacting surface 220 of staple cartridge 32, by at least one attachment pad 240 positioned onto both a proximal end 260 and a distal end 262 of the tissue contacting surface 220. Attachment pads 240 are disposed between the cartridge buttress 500a and the tissue contacting surface 220. Staple cartridge assembly 200 further includes a first outer edge 248a and second outer edge 248b. As shown in FIG. 2, a distal attachment pad 240a is disposed between first outer edge 248a and a distal end of a central longitudinal slot 238 of staple cartridge assembly 200. Similarly, a distal attachment pad 240a is disposed between second outer edge 248b and the distal end of central longitudinal slot 238. Proximal attachment zones 240b are disposed between first outer edge 248a and a proximal end of central longitudinal slot 238 and between second outer edge 248b and the proximal end of central longitudinal slot 238. In this embodiment, central attachment pads 240c are disposed between the proximal end 260 and distal end 262 of tissue contacting surface 220 between the first outer edge 248a and central longitudinal slot 238 and between the second outer edge 248b and central longitudinal slot 238. Other embodiments are contemplated wherein the plurality of attachment pads 240 are disposed in varying positions along tissue contacting surface 220.

With reference to FIG. 3, and similar to cartridge buttress 500a, anvil buttress 500b is operatively secured or adhered to a tissue contacting surface 320 of anvil plate 302 of anvil assembly 300 by at least one attachment pad 340 positioned onto both a proximal end 360 and a distal end 362 of the tissue contacting surface 320. Distal attachment pads 340a are disposed between outer edges 348a, 348b and a distal end of a central longitudinal slot 338. Proximal attachment pads 342b are disposed between outer edges 348a, 348b and a proximal end of the central longitudinal slot 338. Further, central attachment zones 340c are positioned between proximal end 360 and distal end 363 and between outer edges 34a, 348b and central longitudinal slot 338.

The attachment pads can be round, oblong, rectangular, or have any shape and can be provided on at least one of the tissue contacting surfaces of the end effector assembly in any of the embodiments disclosed herein.

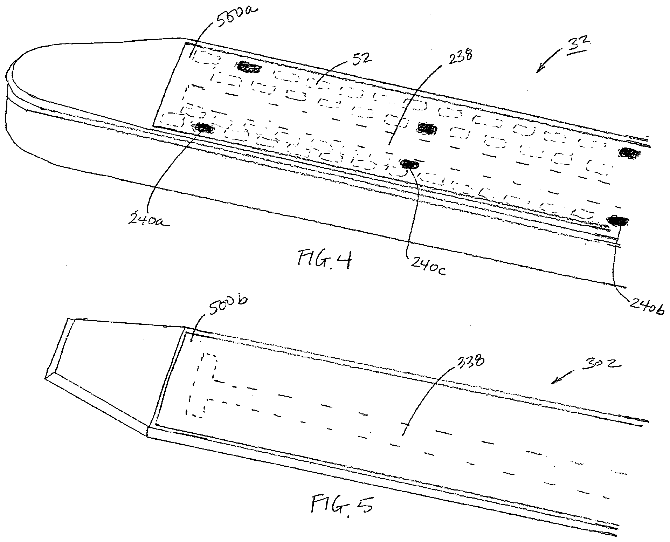

In embodiments, it is contemplated that attachment pads 240, 340 are disposed into staple retaining pockets 52 and staple forming pockets 68 by overmolding, injection molding, or by snap fitting and fixing preformed parts onto the respective tissue contacting surfaces 220, 320. FIGS. 4 and 5 illustrate the buttresses 500a, 500b disposed on the staple cartridge 32 and anvil plate 302, respectively. As shown in FIG. 4, attachment pads 240 may be disposed within selectively positioned staple retaining pockets 52 along tissue contacting surface 220 of staple cartridge 200. Similarly, as shown in FIG. 5, attachment pads 340 are disposed within staple forming pockets 68 along tissue contacting surface 320 of anvil plate 302.

During assembly the buttresses 500a, 500b are placed onto each of the tissue contacting surfaces 220, 320 of staple cartridge assembly 200 and anvil assembly 300, respectively. Ultrasonic welding is used to bond buttresses 500a, 500b to the respective attachment pads 240, 340 and thus to the respective tissue contacting surfaces 220, 320. Other bonding methods are also envisioned such as laser welding, solvent bonding, or heat pressing. As described above, since buttresses 500a, 500b are made from the same material as attachment pads 240, 340, the bonding strength and release forces between the buttresses and attachment pads 240, 340 are balanced so that the buttress material remains secured to the tissue contact surfaces 220, 320 during tissue manipulation and stapler positioning. It is also contemplated that attachment zones or pads can be disposed in the longitudinal slot of the anvil plate, staple cartridge 32, or both, in any of the embodiments disclosed herein.

As illustrated in FIG. 6, during use of surgical stapling apparatus 10, the first jaw or staple cartridge assembly 200 and the second jaw or anvil assembly 300, having surgical buttresses 500a, 500b loaded thereon (as described above) are positioned on either side of the surgical site. Tissue contacting surfaces 220, 320 of staple cartridge assembly 200 and anvil assembly 300 are positioned adjacent layers of tissue "T" to be fastened to one another.

As shown in FIG. 7, staple cartridge assembly 200 includes surgical staples 50 positioned within individual staple retaining slots 52 of staple cartridge 32. Staples 50 are of a conventional type and include a backspan 54 having a pair of legs 56 and 58 extending from backspan 54. Legs 56 and 58 terminate in tissue penetrating tips 60 and 62, respectively. Pushers 64 are located within staple retaining slots 52 and are positioned between staples 50 and the path of a drive bar 36.

In the illustrated embodiment, surgical stapling apparatus 10 is initially actuated by movement of trigger 33 relative to handle 12 (FIG. 1) causing driver 36 to move in the direction of arrow "A" (FIG. 6), and against sloped edge 21 of anvil plate 302 thereby causing anvil assembly 300 to be moved to the closed position relative to staple cartridge assembly 200. As drive bar 36 advances distally within staple cartridge 32, drive bar 66 interacts with a wedge shaped sled 66 to urge the pushers 64 upwardly against backspan 54 of staples 50, driving legs 56 and 58 of staples 50 through the cartridge buttresses 500a, tissue "T", and anvil buttress 500b, towards staple forming pockets or recesses 68 in anvil plate 302 of anvil assembly 300. Tissue penetrating tips 60 and 62 of staple legs 56 and 58 are bent within staple forming pockets 68 in anvil plate 302 with backspan 54 securing surgical buttress 500 against tissue "T".

As shown in the embodiment in FIG. 7, attachment zones 240, 340 are interposed between the respective tissue contacting surfaces 220, 320 of staple cartridge 32 and anvil plate 302 and cartridge buttress 500a and anvil buttress 500b, respectively. Opening end effector assembly 16, after firing, releases the bond between cartridge buttress 500a and attachment zone 240 thereby releasing the cartridge buttress 500a from the tissue contacting surface 220 of the staple cartridge 32. Similarly, the bond between anvil buttress 500b and attachment zone 340 is broken and anvil buttress 500b is released from tissue contacting surface 320 of anvil plate 302. As described above, since buttresses 500a, 500b are made from the same material, there less risk of removing any non-degradable material from either staple cartridge 32 or anvil plate 302 into the body upon release of the bond between surgical buttresses 500a, 500b and staple cartridge 32 and anvil plate 302. It is also contemplated that, in any of the embodiments disclosed herein, the buttress can be made from a different material than the attachment zones or pads and may be biodegradable, bioabsorbable, bio-resorbable or non-bioabsorbable. Attachment pads or zones that are made of biodegradable, bioabsorbable, bio-resorbable, or implantable materials are preferred. In any of the embodiments disclosed herein, the buttress, attachment pads or zones, or both, can be made from materials which would quickly degrade in the body, in response to exposure to water, blood, or the like, which would allow the buttress to separate from the instrument. Starches, sugars, or salts could be used.

It is contemplated that, in any of the embodiments disclosed herein, the buttress and attachment pads or zones are made from the same or similar materials to enable easy welding of the buttress to the same or similar materials. Using the same or similar material for the pads or zones is meant to avoid concern of foreign, non-degradable materials entering the body that may break off of the pads or cartridge during buttress detachment.

Upon full actuation of surgical stapling apparatus 10, a knife 30 (FIG. 7) associated with surgical stapling apparatus 10 and carried by driver 36 may be utilized to cut tissue "T", as well as surgical buttresses 500a, 500b between the rows of now formed staples 50. Upon movement of anvil assembly 300 to the open position, spaced apart from staple cartridge assembly 200, buttresses 500a, 500b are pulled away from respective tissue contacting surfaces 220, 320 of respective staple cartridge assembly 200 and anvil assembly 300.

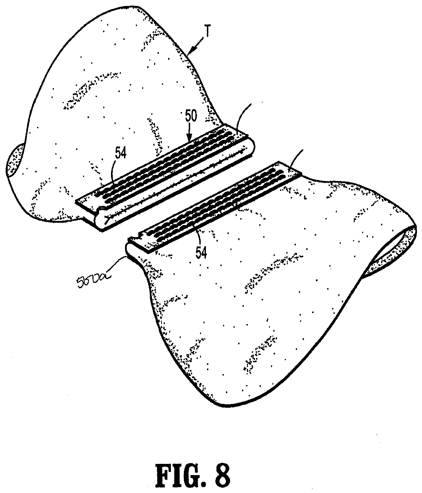

The resulting tissue "T", divided and stapled closed with staples 50, is illustrated in FIG. 8. Specifically, surgical buttresses 500a, 500b are secured against tissue "T" by legs 56, 58 and backspans 54 of staples 50. Thus, surgical buttresses 500a, 500b are stapled to tissue "T" thereby sealing and reinforcing the staple lines created by staples 50.

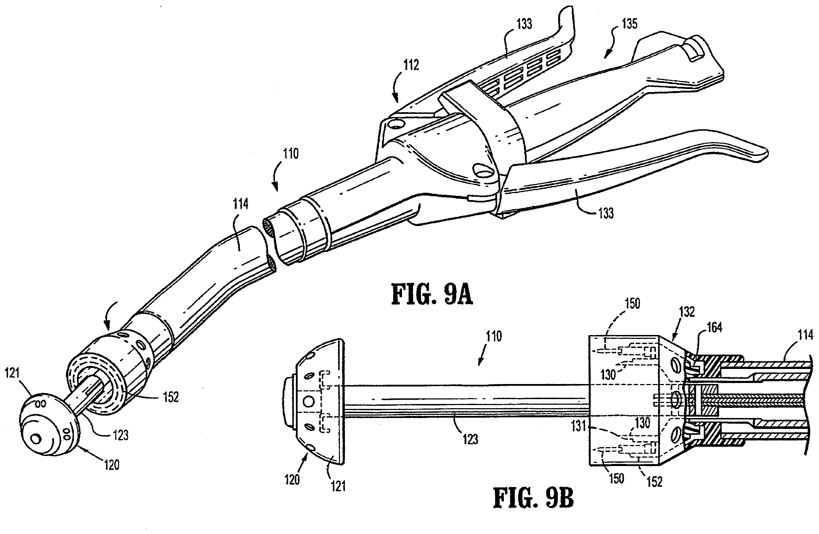

Referring now to FIGS. 9A and 9B, an annular surgical stapling apparatus 110, for use with surgical buttresses 124 of the present disclosure, is shown. Surgical stapling apparatus 110 includes a handle assembly 112 having at least one pivotable actuating handle member 133, and an advancing member 135. Extending from handle member 112, there is provided a tubular body portion 114 which may be constructed so as to have a curved shape along its length. Body portion 114 terminates in a staple cartridge assembly 122 which includes a pair of annular arrays of staple retaining slots 152 having a staple 150 disposed in each one of staple retaining slots 152. Positioned distally of staple cartridge 122 there is provided an anvil assembly 120 including an anvil member 121 and a shaft 123 operatively associated therewith for removably connecting anvil assembly 120 to a distal end portion of stapling apparatus 110.

Staple cartridge assembly 122 may be fixedly connected to the distal end of tubular body portion 114 or may be configured to concentrically fit within the distal end of tubular body portion 114. Staple cartridge assembly 122 includes a staple pusher 164 including a proximal portion having a generally frusto-conical shape and a distal portion defining two concentric rings of peripherally spaced fingers (not shown), each one of which is received within a respective staple retaining slot 152.

A knife 130, substantially in the form of an open cup with the rim thereof defining a knife blade 131, is disposed within staple cartridge assembly 122 and mounted to a distal surface of a staple pusher 164. The knife 130 is disposed radially inward of the pair of annular arrays of staples 150. Accordingly, in use, as the staple pusher 164 is advanced, the knife 130 is also advanced axially distally.

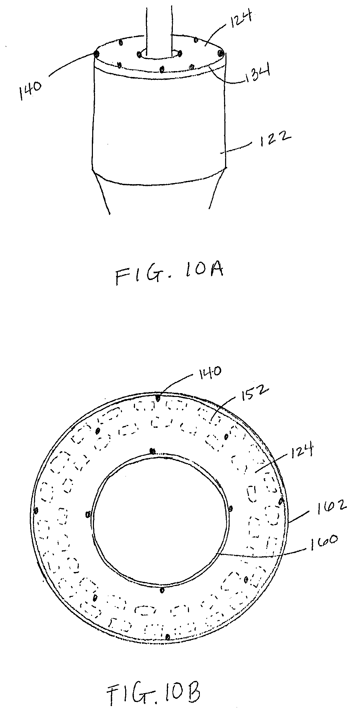

As seen in FIG. 10A, a surgical buttress 124 is releasably attached to the staple cartridge assembly 122 at attachment pads 140 disposed between the surgical buttress 124 and the tissue contacting surface 134 of the staple cartridge assembly 122. As described herein above, attachment pads 140 bond the surgical buttress 124 to the tissue contacting surface 134. Surgical buttress 124 is provided in an annular configuration and includes a central aperture 125 to receive shaft 123 of anvil assembly 120 therethrough.

It is envisioned that the surgical buttress 124 may be additionally or alternatively attached or adhered to tissue contacting surface of anvil plate 121 in a manner similar to the surgical buttress 124 attached to staple cartridge assembly 122.

As shown in FIG. 10B, surgical buttress 124 may be secured or adhered to the staple cartridge 122 along an inner portion or peripheral edge 160 and outer portion or peripheral edge 162 of surgical buttress 124. It is envisioned that other configurations may be utilized to retain the surgical buttress 124 to the staple cartridge assembly 122, such as disposing of the attachment pads 140 within staple retaining retaining slots as discussed with staple cartridge 300, or alternating the attachment zones 140 between the staple retaining slots 152, or among other arrangements within the purview of those skilled in the art.

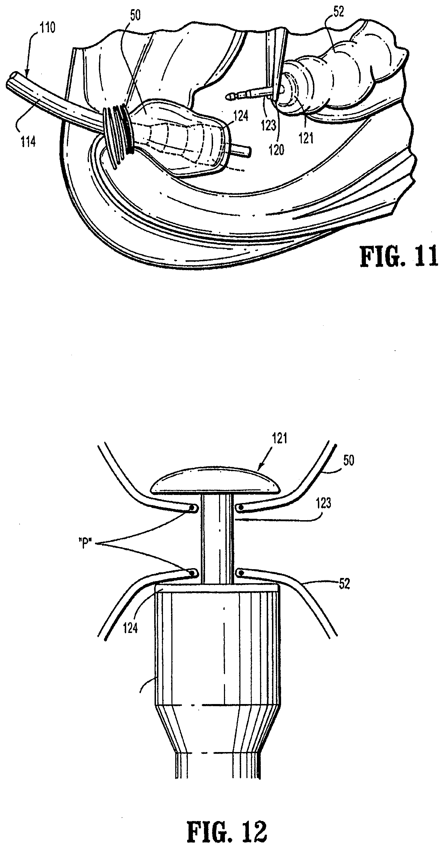

Surgical stapling apparatus 110 and detachable anvil assembly 120 are used in an anastomosis procedure to effect joining of intestinal sections 50 and 52. The anastomosis procedure is typically performed using minimally invasive surgical techniques including laparoscopic means and instrumentation. At the point in the procedure shown in FIG. 11, a diseased intestinal section has been previously removed, anvil assembly120 (optionally including a surgical buttress 124 thereon) has been applied to the operative site either through a surgical incision or transanally and positioned within intestinal section 52, and tubular body portion 114 of surgical stapling apparatus 110 (optionally including a surgical buttress 124 thereon) has been inserted transanally into intestinal section 50. Intestinal sections 50 and 52 are also shown temporarily secured about their respective components (e.g., shaft 123 of anvil assembly 120, and the distal end of tubular body portion 114) by conventional means such as a purse string suture "P", as illustrated in FIG. 12.

Thereafter, the clinician maneuvers anvil assembly 120 until the proximal end of shaft 123 is inserted into the distal end of tubular body portion 114 of surgical stapling apparatus 110, wherein the mounting structure (not shown) within the distal end of tubular body portion 114 engages shaft 123 to effect the mounting. Anvil assembly 120 and tubular body portion 114 are then approximated to approximate intestinal sections 50, 52. Surgical stapling apparatus 110 is then fired. A knife (not shown) cuts the portion of tissue and surgical buttress 124 disposed radially inward of the knife, to complete the anastomosis. The force of the opening of anvil assembly 120 and staple cartridge assembly 122, with surgical buttress 124 stapled to intestinal sections 50 and 52, causes surgical buttress 124 to release at the attachment pads 140 thereby releasing the surgical buttress 124 from the tissue contacting surface 134.

Persons skilled in the art will understand that the devices and methods specifically described herein and illustrated in the accompanying figures are non-limiting exemplary embodiments, and that the description, disclosure, and figures should be construed merely exemplary of particular embodiments. For example it is contemplated that the surgical instrument can be manually or motor-powered in any of the embodiments disclosed herein. It is to be understood, therefore, that the present disclosure is not limited to the precise embodiments described, and that various other changes and modifications may be effected by one skilled in the art without departing from the scope of the disclosure. Additionally, it is envisioned that the elements and features illustrated or described in connection with one exemplary embodiment may be combined with the elements and features of another exemplary embodiment without departing from the scope of the present disclosure, and that such modifications and variations are also intended to be included within the scope of the present disclosure. Accordingly, the subject matter of the present disclosure is not to be limited by what has been particularly shown and described, except as indicated by the appended claims.

## Claims

1. A method for attaching a surgical buttress to an end effector assembly comprising a staple cartridge assembly (200) and an anvil assembly (300), the method comprising:
disposing and securing a plurality of attachment pads (240) within a plurality of staple retaining pockets (52) of a tissue contacting surface (220) of the staple cartridge assembly (200); and/or
disposing and securing a plurality of attachment pads (340) within a plurality of staple forming pockets (68) of a tissue contacting surface (320) of the anvil assembly (300);
placing at least one surgical buttress (500a, 500b) configured and dimensioned to substantially overlie at least a portion of the tissue contacting surface of at least one of the staple cartridge assembly and anvil assembly; and
ultrasonically welding the or each surgical buttress (500a, 500b) to each of the plurality of attachment pads (240, 340).

2. The method of claim 1, wherein at least one of the attachment pads (240, 340) is made from a material selected from the group comprising glycolide, trimethylene carbonate, lactide, caprolactone, and combinations thereof.

3. The method of claim 1 or claim 2, further comprising positioning at least one attachment pad near a central longitudinally extending slot (238, 338) provided in the staple cartridge assembly (200) and the anvil assembly (300) to enable passage of a knife blade therethrough.

4. The method of claim 3, further comprising positioning at least one attachment pad at a distal end (262, 362) of each of the staple cartridge assembly (200) and anvil assembly (300) such that at least one distal attachment pad is positioned between a first outer edge of each of the staple cartridge assembly (200) and anvil assembly (300) and a distal end of the central longitudinally extending slot (238, 338).

5. The method of claim 3 or claim 4, further comprising positioning at least one attachment pad at a proximal end (260, 360) of each of the staple cartridge assembly (200) and anvil assembly (300) such that at least one proximal attachment pad is positioned between a first outer edge of each of the staple cartridge assembly (200) and anvil assembly (300) and a proximal end of the central longitudinally extending slot (238, 338).

6. A staple cartridge (32) for use in a method according to any preceding claims, the staple cartridge (32) comprising:
a cartridge body including a tissue contacting surface (220) defining a plurality of staple retaining slots (52);
a staple (50) disposed within each staple retaining slot (52) of the cartridge body;
a surgical buttress (500a) configured and dimensioned to substantially overlie at least a portion of the staple retaining slots (240) of the cartridge body; **characterised by**
a plurality of attachment pads (240) disposed within staple retaining slots (52) of the cartridge body;
wherein the plurality of attachment pads (240) and the surgical buttress (500a) are made from the same material and the surgical buttress (500a) is ultrasonically welded to each of the plurality of attachment pads (240),
wherein during use the firing force of the staple impacts the attachment pads (240) to break, thereby releasing the surgical buttress (500a) from the tissue contacting surface (220).

## Patentansprüche

1. Ein Verfahren zur Befestigung einer chirurgischen Stütze an einer Endeffektoranordnung, umfassend eine Klammerkassettenanordnung (200) und eine Ambossanordnung (300), wobei das Verfahren Folgendes umfasst:
Anordnen und Sichern mehrerer Befestigungsplatten (240) innerhalb einer Vielzahl von Klammerrückhaltetaschen (52) einer Gewebekontaktoberfläche (220) der Klammerkassettenanordnung (200); und/oder
Anordnen und Sichern einer Vielzahl von Befestigungsplatten (340) innerhalb einer Vielzahl von Klammerbildungstaschen (68) einer Gewebekontaktoberfläche (320) der Ambossanordnung (300);
Anordnen mindestens einer chirurgischen Stütze (500a, 500b), die derart konfiguriert und bemessen ist, dass sie mindestens über einem Teil der Gewebekontaktoberfläche der mindestens einen aus Klammerkassettenanordnung und der Ambossanordnung liegt; und
Ultraschallschweißen der oder jeder chirurgischen Stütze (500a, 500b) an jede der Vielzahl an Befestigungsplatten (240, 340).

2. Verfahren nach Anspruch 1, wobei die mindestens eine der Befestigungsplatten (240, 340) aus einem Material hergestellt ist, das ausgewählt ist aus der Gruppe, umfassend Glykolid, Trimethylencarbonat, Laktid, Caprolacton und Kombinationen davon.

3. Verfahren nach Anspruch 1 oder Anspruch 2, ferner umfassend die Positionierung von mindestens einer Befestigungsplatte nahe einem sich zentral in Längsrichtung erstreckenden Schlitz (238, 338), der in der Klammerkassettenanordnung (200) und der Ambossanordnung (300) zur Verfügung gestellt ist, um den Durchgang einer Messerklinge dort hindurch zu ermöglichen.

4. Verfahren nach Anspruch 3, ferner umfassend das Positionieren mindestens einer Befestigungsplatte an einem distalen Ende (262, 362) von jedem aus Klammerkassettenanordnung (200) und Ambossanordnung (300), derart, dass die mindestens eine distale Befestigungsplatte zwischen einer ersten Außenkante jeder der Klammerkassettenanordnung (200) und Ambossanordnung (300) und einem distalen Ende des sich zentral in Längsrichtung erstreckenden Schlitzes (238, 338) positioniert ist.

5. Verfahren nach Anspruch 3 oder Anspruch 4, ferner umfassend das Positionieren mindestens einer Befestigungsplatte an einem proximalen Ende (260, 360) jeder der Klammerkassettenanordnung (200) und der Ambossanordnung (300), derart, dass die mindestens eine proximale Befestigungsplatte zwischen einer ersten Außenkante jeder der Klammerkassettenanordnung (200) und der Ambossanordnung (300) und einem proximalen Ende des sich mittig in Längsrichtung erstreckenden Schlitzes (238, 338) positioniert ist.

6. Klammerkassette (32) zur Verwendung in einem Verfahren nach einem der vorstehenden Ansprüche, wobei die Klammerkassette (32) Folgendes umfasst:
einen Kassettenkörper, der eine Gewebekontaktoberfläche (220) beinhaltet, die mehrere Klammerrückhalteschlitze (52) definiert;
eine Klammer (50), die innerhalb jedes Klammerrückhalteschlitzes (52) des Kassettenkörpers angeordnet ist;
eine chirurgische Stütze (500a), die derart konfiguriert und bemessen ist, dass sie mindestens über einem Teil der Klammerrückhalteschlitze (240) des Kassettenkörpers liegt; **gekennzeichnet durch**
eine Vielzahl an Befestigungsplatten (240), die innerhalb der Klammerrückhalteschlitze (52) des Kassettenkörpers angeordnet sind;
wobei die Vielzahl an Befestigungsplatten (240) und die chirurgische Stütze (500a) aus demselben Material hergestellt sind und die chirurgische Stütze (500a) an jede der Vielzahl an Befestigungsplatten (240) ultraschallgeschweißt ist,
wobei während der Verwendung die Abschusskraft der Klammer derart auf die Befestigungsplatten (240) einwirkt, dass diese brechen und dabei die chirurgische Stütze (500a) aus der Gewebekontaktoberfläche (220) freigeben.

## Revendications

1. Procédé de fixation d'un support chirurgical à un ensemble effecteur terminal comprenant un ensemble de cartouche d'agrafes (200) et un ensemble d'enclume (300), le procédé comprenant les étapes consistant à :
disposer et fixer une pluralité de pastilles de fixation (240) dans une pluralité de poches de retenue d'agrafes (52) d'une surface (220) de l'ensemble de cartouche d'agrafes (200) en contact avec un tissu ; et/ou
disposer et fixer une pluralité de pastilles de fixation (340) dans une pluralité de poches formatrices d'agrafes (68) d'une surface (320) de l'ensemble d'enclume (300) en contact avec le tissu :
placer au moins un support chirurgical (500a, 500b) configuré et dimensionné pour recouvrir sensiblement au moins une partie de la surface en contact avec le tissu d'au moins l'un de l'ensemble de cartouche d'agrafes et de l'ensemble d'enclume ; et
souder par voie ultrasonique le ou chaque support chirurgical (500a, 500b) à chacune de la pluralité de pastilles de fixation (240, 340).

2. Procédé selon la revendication 1, dans lequel au moins l'une des pastilles de fixation (240, 340) est constituée d'un matériau choisi dans le groupe comprenant un glycolide, du carbonate de triméthylène, un lactide, une caprolactone ou une de leurs combinaisons.

3. Procédé selon la revendication 1 ou la revendication 2, comprenant en outre le positionnement d'au moins une pastille de fixation à proximité d'une fente centrale s'étendant longitudinalement (238, 338) ménagée dans l'ensemble de cartouche d'agrafes (200) et dans l'ensemble d'enclume (300) pour permettre le passage d'une lame de couteau à travers ceux-ci.

4. Procédé selon la revendication 3, comprenant en outre le positionnement d'au moins une pastille de fixation à une extrémité distale (262, 362) de chacun de l'ensemble de cartouche d'agrafes (200) et de l'ensemble d'enclume (300) de sorte qu'au moins une pastille de fixation distale soit positionnée entre un premier bord externe de chacun de l'ensemble de cartouche d'agrafes (200) et de l'ensemble d'enclume (300) et une extrémité distale d'une fente centrale s'étendant longitudinalement (238, 338).

5. Procédé selon la revendication 3 ou la revendication 4, comprenant en outre le positionnement d'au moins une pastille de fixation à une extrémité proximale (260, 360) de chacun de l'ensemble de cartouches d'agrafe (200) et de l'ensemble d'enclume (300) de sorte qu'au moins une pastille de fixation proximale soit positionnée entre un premier bord externe de chacun de l'ensemble de cartouche d'agrafes (200) et de l'ensemble d'enclume (300) et une extrémité proximale de la fente centrale s'étendant longitudinalement (238, 338).

6. Cartouche d'agrafes (32) pour utilisation dans un procédé selon l'une quelconque des revendications précédentes, la cartouche d'agrafes (32) comprenant :
un corps de cartouche comprenant une surface (220) en contact avec le tissu définissant une pluralité de fentes de retenue d'agrafes (52) ;
une agrafe (50) disposée dans chaque fente de retenue d'agrafe (52) du corps de cartouche ;
un support chirurgical (500a) configuré et dimensionné pour recouvrir sensiblement au moins une partie des fentes de retenue d'agrafes (240) du corps de cartouche ; **caractérisée par** :
une pluralité de pastilles de fixation (240) disposées dans des fentes de retenue d'agrafes (52) du corps de cartouche ;
dans laquelle la pluralité de pastilles de fixation (240) et le support chirurgical (500a) sont constituées du même matériau et le support chirurgical (500a) est soudé par voie ultrasonique à chacune de la pluralité de pastilles de fixation (240),
dans laquelle, en cours d'utilisation, la force de frappe de l'agrafe heurte les pastilles de fixation (240) pour les briser, libérant de la sorte le support chirurgical (500a) de la surface (220) venant en contact avec le tissu.
